# EUROPEAN PATENT APPLICATION

(11) **EP 3 610 907 A1**
(43) Date of publication of application: **19.02.2020**
(21) Application number: 18844552.2
(22) Date of filing: 04.01.2018
(51) Int. Cl.: A61M 5/20, A61M 5/24

(54) **RAPID AND AUTOMATIC SYRINGE**

(30) Priority: 09.08.2017 CN 201710675780; 09.08.2017 CN 201720992429 U
(71) Applicant: Gansu Changee Bio-pharmaceutical Co., Ltd., Tianshui, Gansu 741020 (CN)
(72) Inventor: QU, Yanling, Tianshui Gansu 741020 (CN); HAN, Yitao, Tianshui Gansu 741020 (CN); PAN, Junfeng, Tianshui Gansu 741020 (CN); LI, Guoan, Tianshui Gansu 741020 (CN)
(74) Representative: Schollweck, Susanne
(86) International application number: PCT/CN2018/071333
(87) International publication number: WO 2019/029122

(57) **Abstract**

A rapid and automatic syringe comprises a rear casing (5), a front casing (6) connected to the rear casing (5), a sleeve (3) fitted in the rear casing (5), a support (1) fitted in the front casing (6) and abutting a front end surface of the sleeve (3), a cartridge and an injection needle mounted in the support (1), a plunger (2) mounted in the sleeve (3) capable of pushing the cartridge forward, and a trigger member (4) fit as a sleeve between the front casing (6) and the support (1) capable of moving a set distance backward, so as to drive the plunger (2) to move backward. A snap-engaging mechanism capable of retracting inwardly is provided at the rear end of the plunger (2), and an engagement block in an annular groove of the sleeve (3) is capable of being caught in the snap-engaging mechanism. A groove having an inner diameter smaller than the outer diameter of the snap-engaging mechanism is arranged in the rear end surface of the rear casing (5) and is used to retract the snap-engaging mechanism. A driving spring (7) in an initially compressed state is fitted at the periphery of the plunger (2). The front end of the driving spring (7) abuts a protruding ring in the middle of the plunger (2) and the rear end of the driving spring (7) abuts the engagement block. The driving spring (7) releases when the outer diameter of the snap-engaging mechanism is smaller than the inner diameter of the annular groove, such that the plunger (2) is driven to move forward. The rapid and automatic syringe effectively resolves an issue in which it is inconvenient to deliver liquid medicine using a syringe.

## Description

The present application claims the priority to Chinese Patent Application No. 201710675780.3, titled "RAPID AND AUTOMATIC SYRINGE", filed on August 09, 2017 and No.201720992429.2, titled "RAPID AND AUTOMATIC SYRINGE", filed on August 09, 2017 with the China National Intellectual Property Administration, which are incorporated herein by reference in their entireties.

### FIELD

The present application relates to the field of medical supplies, and in particular to a rapid and automatic syringe.

### BACKGROUND

With the improvement of people's living standards, people gradually begin to pay attention to their own health. When an emergency occurs, such as anaphylactic shock or acute attack, injecting drugs before a rescue vehicle coming can reduce the patient's mortality and pain.

In the conventional technology, a pharmaceutical syringe is relatively professional, thus it is not suitable for ordinary people to use and not convenient to operate, people would be hurt by faulty operation, and the pharmaceutical syringe is not convenient to use and not convenient to carry around; before injection, a syringe needle is exposed to the outside, increasing the patient's fear, after the injection is completed, the syringe needle cannot be bounced back, thus there is a hidden danger of safety. It may be operated by a single person.

In summary, how to effectively solve the problem that the pharmaceutical syringe is inconvenient in use is a problem presently to be solved by those skilled in the art.

### SUMMARY

An object of the present application is to provide a rapid and automatic syringe, which effectively solves the problem that the pharmaceutical syringe is inconvenient to use.

In order to solve the above technical problem, following technical solutions are provided by the present application:

A rapid and automatic syringe, which includes a rear shell, a front shell connected to the rear shell, a sleeve sleeved in the rear shell, a bracket sleeved in the front shell and abutting against a front end surface of the sleeve, a cartridge bottle and an injection needle mounted in the bracket, a push rod mounted in the sleeve and capable of pushing the cartridge bottle to move forward, and a trigger member sleeved between the front shell and the bracket and capable of moving backward a set distance to drive the push rod to move backward.

A rear end of the push rod has a snap-engaging mechanism capable of contracting inwardly, an engagement block in the annular groove of the sleeve is capable of being stuck in the snap-engaging mechanism, a groove having an inner diameter smaller than an outer diameter of the snap-engaging mechanism is arranged in a rear end surface of the rear shell and is used for contracting the snap-engaging mechanism, a drive spring in an initially compressed state is mounted at a periphery of the push rod, a front end of the drive spring abuts a middle step ring of the push rod and the rear end of the drive spring abuts the engagement block, the drive spring is released when the outer diameter of the snap-engaging mechanism is smaller than the inner diameter of the annular groove, such that the push rod is driven to move forward.

Preferably, the rear end of the trigger member has a hook capable of contracting inwardly, and the periphery of the trigger member is provided with a callback spring, the front end of the callback spring abuts a middle protruding ring of the trigger member and the rear end abuts a snap ring, the snap ring is fixed to the rear end surface of the front shell, and the snap ring can be stuck in the hook.

Preferably, the rear end surface of the bracket has a stepped surface, and there is a gap between the hook of the trigger member and the stepped surface, after the trigger member is triggered, the rear end surface of the hook can abuts the stepped surface.

Preferably, the rapid and automatic syringe further includes a protective cap mounted at the rear shell for encapsulating the groove, and a core of the protective cap is inserted into a limit groove in the snap-engaging mechanism.

Preferably, the rapid and automatic syringe further includes a cushion mounted at the front end of the front shell.

Preferably, the rapid and automatic syringe further includes a sealing ring mounted between the rear shell and the front shell for sealing the injection needle inside the rapid and automatic syringe.

Preferably, the rear shell and the front shell are connected by a thread or by the snap-engaging mechanism.

Preferably, the cartridge bottle is a transparent bottle, a visual window is arranged on the front shell and the bracket for checking whether the medicine solution in the cartridge bottle is injected completely or not.

The rapid and automatic syringe provided by the present application includes the rear shell, the front shell, the sleeve, the bracket, the push rod and the trigger member. The front shell is connected with the rear shell, which is an outer shell of the rapid and automatic syringe and used for fixing the internal parts. The sleeve is nested in the rear shell, a positional movement of the sleeve in an axial direction is limited by the rear shell. The bracket is sleeved in the front shell and abuts against the front end face of the sleeve to transfer an axial force. It should be noted that the term "front" described here or below refers to a side close to an injection position, and the term "rear" refers to a side far away from the injection position. The cartridge bottle and the injection needle are mounted in the bracket, and the bracket limits a radial movement of the cartridge bottle; preferably, the cartridge bottle and the injection needle are separated to avoid drug leakage and contamination. The sleeve is connected with the snap-engaging mechanism of the bracket to fix the cartridge bottle; the drive spring is positioned by the sleeve and the push rod together, so that the drive spring is in a compressed state before being released.

The push rod is mounted in the sleeve and can push the cartridge bottle to move forward. The trigger member is mounted between the front shell and the bracket, and can move in the axial direction. The trigger member is pressed in the injection position and a certain force is applied, thus the trigger member moves backward the set distance to drive the push rod to move backward. The rear end of the push rod has a snap-engaging mechanism capable of contracting inward, the rear end of the sleeve has an annular groove, the rear end surface of the rear shell has a groove hole, and the inner diameter of the groove hole is less than the outer diameter of the snap-engaging mechanism, which is used for contracting the snap-engaging mechanism. The engagement block of the annular groove can be stuck in the snap-engaging mechanism, after the push rod moves backward the set distance and reaches the groove hole, the groove hole makes the snap-engaging mechanism contract, the snap-engaging mechanism at the rear end is separated from the engagement block, and the snap-engaging mechanism contracts. A drive spring in an initially compressed state is sleeved on the periphery of the push rod, and the front end of the drive spring abuts a protruding ring in the middle of the push rod, and the rear end abuts the engagement block. The drive spring releases when the outer diameter of the snap-engaging mechanism is smaller than the inner diameter of the annular groove, such that the push rod is driven to move forward, thereby pushing the cartridge bottle to move forward, starting to deliver liquid medicine and finishing delivering liquid medicine by syringe.

The rapid and automatic syringe provided by the present application is a one-time first aid pen, the cartridge bottle with drug and a single-use aseptic double-edged injection needle are installed into the rapid and automatic syringe. By using a snap-engaging mechanism lock and a trigger spring technology, the driving spring is locked in the sleeve by means of the snap-engaging mechanism, the spring force is released by triggering the trigger snap-engaging mechanism, such that the push rod is driven to move, and the needle injection and medicine injection are completed through a number of coupling members. When the rapid and automatic syringe is used, the trigger member is pressed to the injection position and a certain pressure is applied, the needle injection and medicine injection can be completed automatically. When an emergency occurs, such as anaphylactic shock or acute attack, ordinary people can complete the injection before a rescue vehicle coming, which can reduce patient's mortality and pain, and the injection can be operated by a single person, which is simple to operate and easy to use, portable, safe and reliable; before injection is performed, the needle is hidden to reduce the fear of patients; the injection process is a fast- needle injection and slow drug injection operation, which is simple and convenient.

### BRIEF DESCRIPTION OF THE DRAWINGS

In order to illustrate more clearly the embodiments of the present application or the technical solutions in the conventional technology, the drawings to be used in descriptions of the embodiments or the conventional technical will be briefly described below. Apparently, the drawings described below are only some embodiments of the present application, and for ordinary technicians in the field, other drawings can be obtained according to these drawings without any creative work for those skilled in the art.
Figure 1 is a schematic structural view of a rapid and automatic syringe according to a specific embodiment of the present application.
Figure 2 is a sectional view of A/A in Figure 1.
Figure 3 is a partial enlarged view of B in Figure 2.
Figure 4 is a partial enlarged view of C in Figure 2.
Figure 5 is an exploded view of the components in Figure 1.

The following are the reference numbers in figures:

| | |
|---|---|
| 1 bracket, | 2 push rod, |
| 3 sleeve, | 4 trigger member, |
| 5 rear shell, | 6 front shell, |
| 7 drive spring, | 8 callback spring, |
| 9 sealing ring, | 10 protective cap, |
| 11 cushion, | 12 snap ring |

### DETAILED DESCRIPTION OF EMBODIMENTS

A core of the present application is to provide a rapid and automatic syringe, and the rapid and automatic syringe effectively resolves an issue of a syringe being inconvenient to use.

The technical solutions in the embodiments of the present application will be clearly and fully described below in conjunction with the drawings in the embodiments of the present application. Apparently, the embodiments described are only part of the embodiments of the present application, not all of the embodiments. Based on the embodiments in the present application, all other embodiments obtained by those skilled in the art without creative work fall within the scope of protection of the present application.

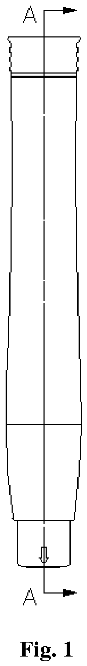

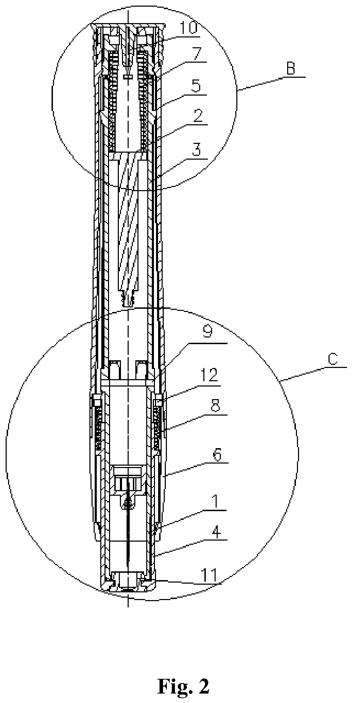

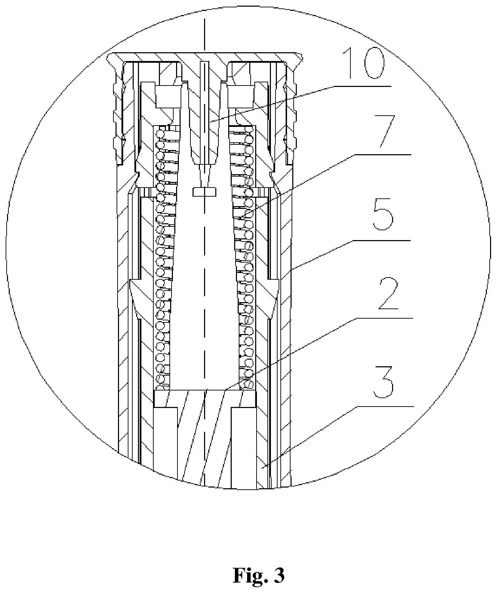

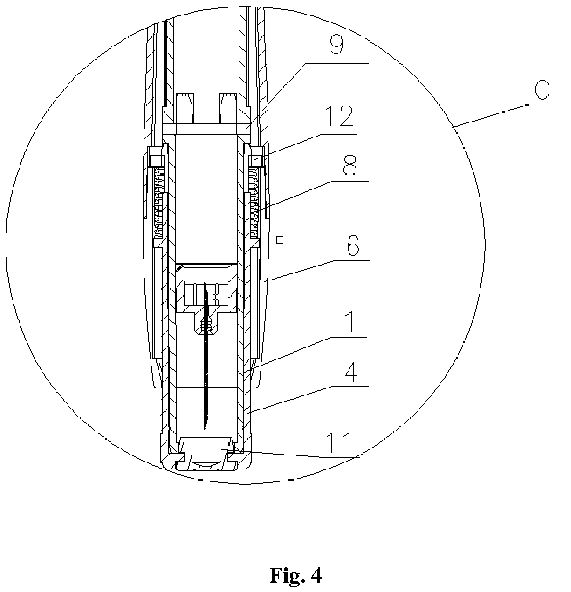

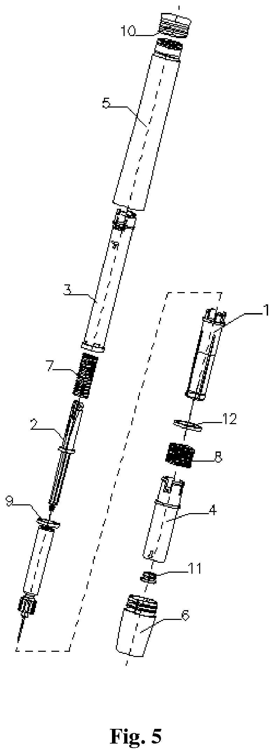

Refer to Figures 1 to 5, Figure 1 is a schematic structural view of the rapid and automatic syringe provided in a specific embodiment of the present application; Figure 2 is a section view of the rapid and automatic syringe taken along A/A in Figure 1; Figure 3 is a partial enlarged view of B in Figure 2; Figure 4 is a partial enlarged view of C in Figure 2; and Figure 5 is an exploded view of the components in Figure 1.

In a specific embodiment, the rapid and automatic syringe provided in the present application includes a rear shell 5, a front shell 6, a sleeve 3, a bracket 1, a push rod 2 and a trigger member 4. The front shell 6 is connected with the rear shell 5 to form an outer shell of the rapid and automatic syringe, which is used for mounting the internal parts. The sleeve 3 is nested in the rear shell 5, and the positional movement of the sleeve 3 in an axial direction is limited by the rear shell 5. The bracket 1 is nested in the front shell 6 and abuts against to a front end face of the sleeve 3, so that an axial force can be delivered. It should be noted that the term "front" described here or below refers to a side close to an injection position, and the term "rear" refers to a side far away from the injection position. A cartridge bottle and a injection needle are mounted in the bracket 1, and the bracket 1 limits the radial movement of the cartridge bottle; preferably, the cartridge bottle and the injection needle are separated, so as to avoid drug leakage and contamination. The sleeve 3 is in a snap-connection with the bracket 1 to secure the cartridge bottle; the drive spring 7 is positioned by the sleeve 3 and the push rod 2 together, so that the drive spring 7 is in a compressed state before being released.

The push rod 2 is mounted in the sleeve 3 and can push the cartridge bottle to move forward. The trigger member 4 is nested between the front shell 6 and the bracket 1, and can move in the axial direction. The trigger member 4 is pressed at the injection position, and a certain force is applied, the trigger member 4 moves backward by a set distance and drives the push rod 2 to move backward. A rear end of the push rod 2 has a snap-engaging mechanism capable of contracting inward, a rear end of the sleeve 3 has an annular groove, a rear end surface of the rear shell 5 has a groove hole, and an inner diameter of the groove hole is less than an outer diameter of the snap-engaging mechanism, which is used for contracting the snap-engaging mechanism. An engagement block of the annular groove can be stuck in the snap-engaging mechanism, and after the push rod 2 moves backward by the set distance and reaches the groove hole, the groove hole makes the snap-engaging mechanism contract, the snap-engaging mechanism at the rear end is separated from the engagement block, and the snap-engaging mechanism contracts. A drive spring 7 in an initially compressed state is mounted on the periphery of the push rod 2, and a front end of the drive spring 7 abuts a step ring in the middle of the push rod 2, and a rear end of the drive spring 7 abuts the engagement block. When the outer diameter of the snap-engaging mechanism is smaller than the inner diameter of the annular groove, the drive spring 7 is released such that the push rod 2 is driven to move forward, thereby the cartridge bottle is pushed to move forward, which and liquid medicine is started to be delivered and delivering liquid medicine by syringe is finished.

The rapid and automatic syringe provided by the present application is a one-time first aid pen, and the cartridge bottle with medicine and a single-use aseptic double-edged injection needle are installed into the rapid and automatic syringe. By using a snap-engaging mechanism lock and a trigger spring technology, the drive spring 7 is locked in the sleeve 3 by means of the snap-engaging mechanism, the spring force is released by triggering the trigger snap-engaging mechanism, such that the push rod 2 is driven to move, and the needle injection and medicine injection are completed through a number of coupling members. When the rapid and automatic syringe is used, the trigger member is pressed to the injection site and a certain pressure is applied, the needle injection and medicine injection can be completed automatically. When an emergency occurs, such as anaphylactic shock or acute attack, ordinary people can complete the injection before a rescue vehicle coming, which can reduce patient's mortality and pain, can be operated by a single person, which is simple to operate and easy to use, which can be portable, safe and reliable; before injection, the needle is hidden to reduce the fear of patients; the injection process is fast- needle injection slow drug injection, which is simple and convenient.

The above rapid and automatic syringe is simply a preferred solution, and is not limited thereto. On this basis, different embodiments can be obtained by targeted adjustments according to actual needs. The rear end of the trigger member 4 has a hook, the hook is capable of contracting inwardly, and a callback spring 8 is sleeved at the periphery of the trigger member 4, and the initial state of the callback spring 8 is compressed. A front end of the callback spring 8 abuts the protruding ring in the middle of the trigger member 4, and a rear end of the callback spring 8 abuts the snap ring 12, the snap ring 12 is fixed to a rear end surface of the front shell 6, the position of the hook is fixed, and the snap ring 12 can be stuck in the hook. Before triggering, the snap ring 12 secures the callback spring 8 to the rear end of the trigger member 4, and the callback spring 8 and the snap ring 12 are limited by the trigger member 4. After the trigger member 4 is pressed, the trigger member 4 moves backward, when the hook at the rear end of the trigger member 4 passes through the rear shell 5, the hook contracts inwardly due to a large outer diameter. When the force applied on the trigger member 4 is removed, and an outer diameter of the hook is less than an inner diameter of the snap ring 12, the callback spring 8 is released, such that the trigger member 4 is pushed forward, at this time, the injection needle is retracted and hidden. The whole injection process is a hidden needle operation, and the needle retracts automatically after the injection, which effectively prevents the accidental puncture injury and reduces the fear and psychological pressure of the users.

On the basis of the above specific embodiment, the rapid and automatic syringe can be modified by those skilled in the art according to the specific occasions, a rear end surface of the bracket 1 has a stepped surface, and there is a gap between the hook and the stepped surface of the trigger member 4. After the trigger member 4 is triggered, a rear end surface of the hook can abuts the stepped surface, the bracket 1 moves forward by a front end inner surface of the trigger member 4 abutting the front end surface of the trigger member 4, which is easy to deliver a driving force, and the structure is simple and compact.

Apparently, under the guidance of this idea, the above specific embodiments can be modified by those skilled in the art according to the specific occasions, a protective cap 10 is further included. The protective cap is mounted in the rear shell 5 for encapsulating the groove hole, and a core portion of the protective cap 10 is inserted in the limit groove in the snap-engaging mechanism. Before using, the protective cap 10 buckles to the outer shell, and the core portion is inserted in the groove hole of the push rod 2, which makes sure that the snap-engaging mechanism of the push rod 2 cannot be retracted until the protective cap 10 is removed, so that the drive spring 7 cannot be released to move. The protective cap 10 can prevent a misoperation, and the protective cap 10 should be removed during injection, so that the trigger member 4 can be pressed, which is relatively safe.

It should be particularly noted that the rapid and automatic syringe provided in the present application should not be limited to this situation, and a cushion 11 is further included. The cushion is mounted at the front end of the front shell 6 and the front end of the bracket 1. After the drive spring 7 is released, the cushion is used for buffering the injection needle, cartridge bottle and the like when the injection needle, cartridge bottle and the like are pushed forward, so as to prevent the bottle from breaking due to strong collision.

The rapid and automatic syringe provided in the present application further includes a sealing ring 9, with other components being unchanged. The sealing ring 9 is mounted between the rear shell 5 and the front shell 6, which is used for sealing the injection needle inside the rapid and automatic syringe, thereby preventing needle contamination, ensuring aseptic, which is relatively safe and hygienic.

For the rapid and automatic syringe in the above embodiments, the rear shell 5 and the front shell 6 are in a thread connection, which is easy to connect, simple in thread processing, detachable connection, convenient to disassemble and convenient to use. The rear shell 5 and the front shell 6 can also be connected by the snap-engaging mechanism, whose structure is simple, one-time use, and disassembly is prevented.

In order to further optimize the above technical solution, the cartridge bottle is a transparent bottle. A visual window is arranged on the front shell and the bracket for checking whether the medicine solution in the cartridge bottle is injected completely or not, which is more convenient to use.

In the description of the present application, it is to be noted that the orientation or positional relationship indicated by the terminology, such as front and rear, is based on the orientation or positional relationship shown in the drawings, which is only to facilitate the description of the present application and to simplify the description, rather than indicating or implying that the device or element referred to must have a specific orientation, construct and operate in a particular orientation, and therefore cannot be understood as a restriction on the present application.

The embodiments in the present specification are described in a progressive manner, each of which focuses on differences from other embodiments, and the same or similar parts of each embodiment can be referred to each other.

The above description of the disclosed embodiment enables those skilled in the art to realize or use the present application. Various modifications to these embodiments are apparent to those skilled in the art, and the general principles defined herein may be implemented in other embodiments without departing from the spirit or scope of the present application. Therefore, the present application is not limited to these embodiments shown herein, but will be consistent with the widest range of principles and novel features disclosed herein.

## Claims

1. A rapid and automatic syringe, **characterized in that**, the rapid and automatic syringe comprises a rear shell (5), a front shell (6) connected to the rear shell (5), a sleeve (3) nested in the rear shell (5), a bracket (1) nested in the front shell (6) and abutting against a front end surface of the sleeve (3), a cartridge bottle and an injection needle mounted in the bracket (1), a push rod (2) mounted in the sleeve (3) and configured to push the cartridge bottle to move forward, and a trigger member (4) mounted between the front shell (6) and the bracket (1) and configured to move backward a set distance to drive the push rod (2) to move backward,
a rear end of the push rod (2) has a snap-engaging mechanism contractible inwardly, an engagement block in an annular groove of the sleeve is allowed to be stuck in the snap-engaging mechanism, a groove having an inner diameter smaller than an outer diameter of the snap-engaging mechanism is arranged in a rear end surface of the rear shell (5) and is configured to contract the snap-engaging mechanism, a drive spring (7) in an initially compressed state is sleeved at a periphery of the push rod (2), a front end of the drive spring (7) abuts a middle step ring of the push rod (2) and the rear end of the drive spring (7) abuts the engagement block, the drive spring (7) is released when the outer diameter of the snap-engaging mechanism is smaller than the inner diameter of the annular groove, which allows the push rod (2) is driven to move forward.

2. The rapid and automatic syringe according to claim 1, **characterized in that**, the rear end of the trigger member (4) has a hook contractible inwardly, and a periphery of the trigger member (4) is provided with a callback spring (8), the front end of the callback spring (8) abuts a middle protruding ring of the trigger member (4) and the rear end abuts a snap ring (12), the snap ring (12) is fixed to the rear end surface of the front shell (6), and the snap ring (12) is allowed to be stuck in the hook.

3. The rapid and automatic syringe according to claim 2, **characterized in that**, a rear end surface of the bracket (1) has a stepped surface, and a gap is present between the hook of the trigger member (4) and the stepped surface, after the trigger member (4) is triggered, a rear end surface of the hook is allowed to abut the stepped surface.

4. The rapid and automatic syringe according to any of claims 1 to 3, **characterized in that**, the rapid and automatic syringe further comprises a protective cap (10) mounted at the rear shell (5) for encapsulating the groove, and a core of the protective cap (10) is inserted into a limit groove in the snap-engaging mechanism.

5. The rapid and automatic syringe according to claim 4, **characterized in that**, the rapid and automatic syringe further comprises a cushion (11) mounted at the front end of the front shell (6).

6. The rapid and automatic syringe according to claim 4, **characterized in that**, the rapid and automatic syringe further comprises a sealing ring (9) mounted between the rear shell (5) and the front shell (6) for sealing the injection needle inside the rapid and automatic syringe.

7. The rapid and automatic syringe according to claim 4, **characterized in that**, the rear shell (5) and the front shell (6) are connected by a thread or by another snap-engaging mechanism.

8. The rapid and automatic syringe according to claim 4, **characterized in that**, the cartridge bottle is a transparent bottle, a visual window is arranged on the front shell (6) and the bracket (1) for checking whether the medicine solution in the cartridge bottle is injected completely or not.
